# EUROPEAN PATENT APPLICATION

(11) **EP 1 752 157 A1**
(43) Date of publication of application: **14.02.2007**
(21) Application number: 06118247.3
(22) Date of filing: 01.08.2006
(51) Int. Cl.: A61K 36/185, A61K 31/05, A61K 31/22, A23L 1/30

(54) **Prevention and treatment of atherosclerosis with lithospermate B**

(30) Priority: 01.08.2005 US 193355
(71) Applicant: Industry-Academic Cooperation Foundation, Seodaemun-gu Seoul 120-749 (KR)
(72) Inventor: Lee, Hyun Chul, Sinchon-dong Seodaemun-gu Seoul 120-752 (KR); Jung, Mankil, Seodaemun-gu Seoul 120-749 (KR); Hur, Kyu Yeon, Buk-gu Gwangju, 500-712 (KR)
(74) Representative: Behnisch, Werner

(57) **Abstract**

This invention relates to lithospermate B ('LAB' hereinafter) that inhibits proliferation and migration of vascular smooth muscle cells ('VSMC' hereinafter). More specifically, this invention relates to a pharmaceutical composition comprising LAB isolated from *Salviae miltiorrhiza* as an active ingredient for treating atherosclerosis.

## Description

### TECHNICAL FIELD OF THE INVENTION

This invention relates to lithospermate B ('LAB' hereinafter) that inhibits proliferation and migration of vascular smooth muscle cells ('VSMC' hereinafter). More specifically, this invention relates to a pharmaceutical composition comprising LAB isolated from *Salviae miltiorrhiza* as an active ingredient for treating atherosclerosis.

### BACKGROUND OF THE INVENTION

Generally, atherosclerosis is known as a major cause of cardiac infarction, cerebral hemorrhage, and peripheral vascular diseases and it accounts for more than half of the deaths in advanced countries. According to the report from American Heart Association, for example, [Ross R. The pathogenesis of atherosclerosis: a perspective for the 1990s. Nature. Apr. 29, 1993; 362 (6423): 801-809], about 500,000 people die of cardiac infarction every year while about 150,000 people die of cerebral infarction in U.S.

'VSMC', being one of the major components that forms blood vessel wall, serves to maintain the shape and the physiological activities of blood vessels [Vinters HV, Berliner JA. The blood vessel wall as an insulin target tissue. Diabete Metab. Jul 1987;13(3 Pt 2):294-300]. In addition, the quantitative and qualitative increase of 'VSMC' as well as its migration play a key role in developing atherosclerosis.

Various factors such as growth factor, oxidation stress, physical shear and the like are associated with the development of atherosclerosis. Platelet-derived growth factor is a dimer connected by a disulfide bond and binds tyrosine kinase receptor, which is known to be involved in various cellular functions including gene expression, growth, differentiation, and migration of cells and the like.

It has been already known that platelet-derived growth factor enables to proliferate various kinds of cells, in particular, vascular smooth muscle cells. Therefore, numerous researches have been conducted to find ways to prevent atherosclerosis by inhibiting the proliferation of 'VSMC' by means of platelet-derived growth factor [Nishio E, Watanabe Y. Homocysteine as a modulator of platelet-derived growth factor action in vascular smooth muscle cells: a possible role for hydrogen peroxide. Br J Pharmacol. Sep 1997;122(2):269-274].

Further, growth factors are known to increase the level of reactive oxygen species ('ROS' hereinafter) in the 'VSMC' [Sundaresan M, Yu ZX, Ferrans VJ, et al. Requirement for generation of H2O2 for platelet-derived growth factor signal transduction. Science. Oct 13 1995;270(5234):296-299].

'RSO' is very unstable and also highly reactive so that it induces an irreversible chemical reaction to polymers such as proteins and lipids. These harmful chemical reactions deteriorate cellular functions and cause cell apoptosis [Takano H, Zou Y, Hasegawa H, et al. Oxidative stress-induced signal transduction pathways in cardiac myocytes: involvement of ROS in heart diseases. Antioxid Redox Signal. Dec 2003;5(6):789-794].

ROS such as superoxide anion (O₂⁻) or hydrogen peroxide (H₂O₂) is known as a product of cellular respiration and there have been many reports recently disclosing that the ROS is involved in cellular signal transduction in mammals [Finkel T. Oxygen radicals and signaling. Curr Opin Cell Biol. Apr 1998;10(2):248-253].

Growth factors such as ROS or PDGF-BB are known to activate signal transduction pathways such as Raf-MEK-MAPK and phosphatidyl inositol 4,5-bisphosphate 3-kinase(PI3K)-Akt. For example, p42/44 extracellular-regulated kinase(ERK 1/2), as one of MAPK families, is a well known modulator of signal transduction in 'VSMC' [Graves LM, Bomfeldt KE, Sidhu JS, et al. Platelet-derived growth factor stimulates protein kinase A through a mitogen-activated protein kinase-dependent pathway in human arterial smooth muscle cells. J Biol Chem. Jan 5 1996;271(1):505-511; Force T, Bonventre JV. Growth factors and mitogen-activated protein kinases. Hypertension. Jan 1998;31(1 Pt 2):152-161.].

The activation of these signal transduction modulators leads to phosphorylation of many proteins in cell wall or cytoplasm thereby regulating transcription of genes. For example, the signal transduction pathways of Raf, MAPK kinase 1/2(MEK-1/2) and ERK1/2 are also regulated by the phosphorylation. The activation of ERK1/2 is a very critical modulator in signal transduction of growth factors and is also essential for the proliferation and migration of 'VSMC'. Besides, ROS and PDGF-BB are also related to other signal transduction modulators, that is, other signal transduction pathway related to cell vitality as in the PI3K and Akt.

MAPK or Akt transduction pathways have been known as two independent modulators having different effects. However, recent reports suggested that there may be present a cross-talk between the two pathways [Guan KL, Figueroa C, Brtva TR, et al. Negative regulation of the serine/threonine kinase B-Raf by Akt. J Biol Chem. Sep 1 2000;275(35):27354-27359; Zimmermann S, Moelling K. Phosphorylation and regulation of Raf by Akt(protein kinase B). Science. Nov 26 1999;286(5445):1741-1744; Rommel C, Clarke BA, Zimmermann S, et al. Differentiation stage-specific inhibition of the Raf-MEK-ERK pathway by Akt. Science. Nov 26 1999;286(5445):1738-1741.].

Radix *Salviae miltiorrhizae,* known as a sage plant in China, is a publicly known herbal medicine that can improve blood circulation and reduce edema. It has been also reported to have effects of vasodilation, antihypertension, anti-coagulation and antibacterial activities. In addition, it has been also reported that it is effective in improving kidney functions in rats with chronic kidney failure.

Recently, the inventors of the present invention succeeded in isolating Lithospermate B, a major substance exhibiting the above-mentioned functions, *S. miltiorrhizae.* The inventors have previously reported that the strong antioxidizing effect of LAB improves the nephrotic in diabetic nephritic rats by reducing ROS [Lee GT, Ha H, Jung M, et al. Delayed treatment with lithospermate B attenuates experimental diabetic renal injury. J Am Soc Nephrol. Mar 2003;14(3):709-720; Yokozawa T, Oura H, Lee TW, et al. Augmentation of renal response by magnesium lithospermate B. Nephron. 1991;57(1):78-83; Fung KP, Wu J, Zeng LH, et al. Lithospermic acid B as an antioxidant-based protector of cultured ventricular myocytes and aortic endothelial cells of rabbits. Life Sci. 1993;53(12):PL189-193; Chung HY, Yokozawa T, Oura H. Effect of extract from Salviae miltiorrhizae radix on uremic rats. Chem Pharm Bull (Tokyo). Sep 1986;34(9):3818-3822].

### SUMMARY OF THE INVENTION

The inventors of the present invention discovered that LAB has such a strong anti-oxidizing activity to prevent vascular smooth muscle tissues from proliferation and migration thereby preventing and treating atherosclerosis. Therefore, the object of this invention is to provide a pharmaceutical composition for proliferation and migration of vascular smooth muscle tissues comprising LAB as an active ingredient to prevent and treat atherosclerosis.

### BRIEF DESCRIPTION OF THE DRAWINGS

The above and other objects and features of the present invention will become apparent from the following description of the invention, when taken in conjunction with the accompanying drawings, wherein:

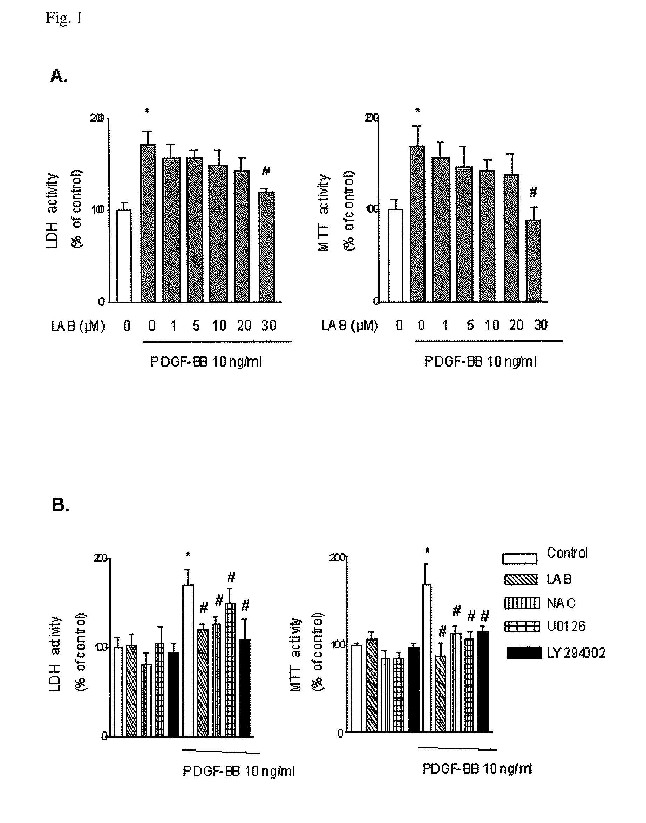

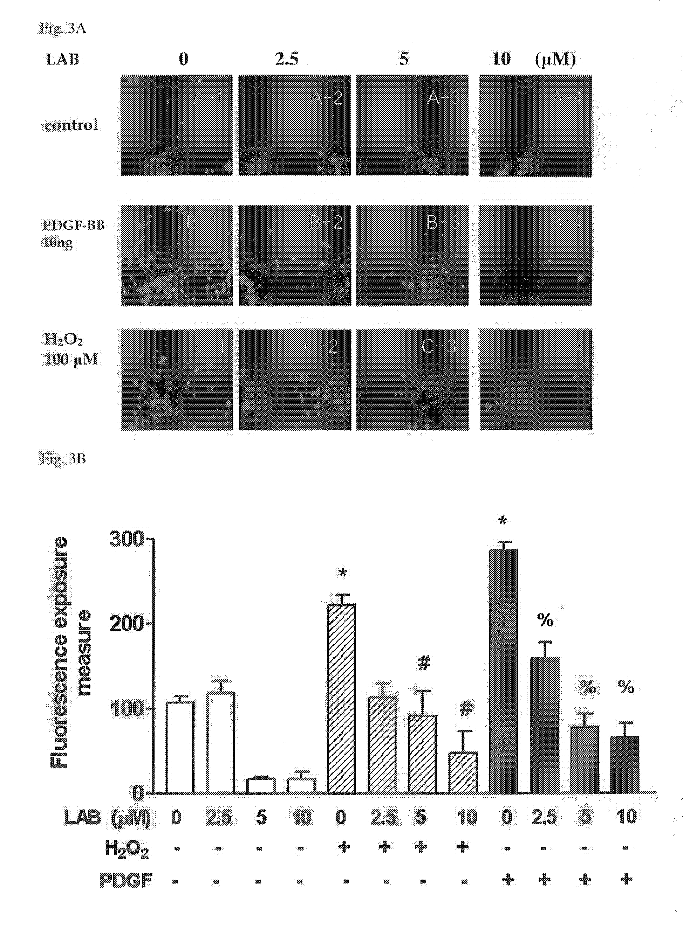

Figs. 1A-1B respectively show the effects of LAB and other various inhibitors in signal transduction pathway on proliferation of vascular smooth muscle cells due to PDGF-BB [1A: VSMCs were incubated in the absence or presence of varying doses of LAB for 1 h and stimulated with 10 ng/ml of PDGF-BB for an additional 24 h. 1B: VSMCs were incubated in the absence or presence of 10µM U0126 (MEK inhibitor), 40µM LY294002 (PI3K inhibitor) and 20 mM N-acetylcysteine (NAC) for 1 h and stimulated with 10 ng/ml PDGF-BB for an additional 24 h. After then, the number of living cells were assessed using LDH and MTT assay. Values are means ± SD. *, *p*< 0.001 compared with the control; #, *p*< 0.001 compared with the presence of PDGF-BB alone treated group (n=18)];

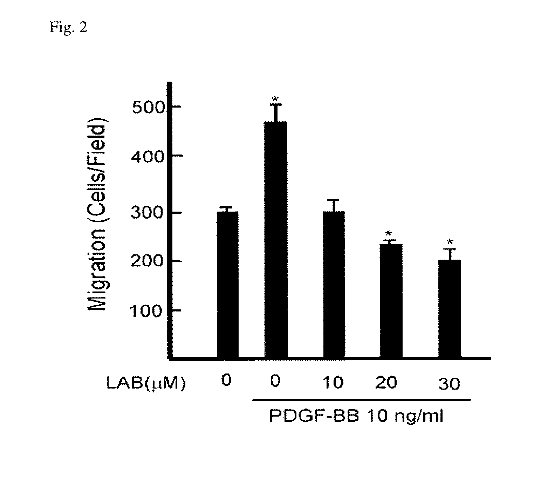

Fig. 2 is a graph showing the effect of LAB on migration of VSMC [16h migration assays were performed using Transwell cell culture chambers seeded with 3.0 x 10⁵ cells per well and PDGF-BB (10 ng/ml) as the chemoattractant. Cells on the outer side of filter or on plates were counted in the eight randomly chosen fields of duplicated filters at x200 magnification. Each column represents the mean ± s.e. (n=6). * *p*<0.05, when compared to the control value.];
First row (A) of Fig. 3 shows the effect of LAB on intracellular ROS levels in VSMCs (A-1: Control, A-2: 2.5 µM LAB, A-3: 5 µM LAB, A-4: 10 µM LAB);
Second row (B) of Fig. 3 shows the effect of LAB on intracellular ROS levels induced by 10 ng/mL PDGF-BB (B-1: PDGF-BB, B-2: PDGF-BB + 2.5 µM LAB, B-3: PDGF-BB + 5 µM LAB, B-4: PDGF-BB + 10 µM LAB);
Third row (C) of Fig. 3 shows the effect of LAB on intracellular ROS levels induced by 100 µM H₂O₂ (C-1: H₂O₂, C-2: H₂O₂ + 2.5 µM LAB, C-3: H₂O₂ + 5 µM LAB, C-4: H₂O₂ + 10 µM LAB)
Fig. 3B shows Values that mean ± SD. *, *p*< 0.001 compared with the control; #, *p*< 0.001 compared with PDGF-BB alone treated group; %, *p*< 0.001 compared with H₂O₂ alone treated group (n=5)],
(more particularly, VSMCs were incubated in the absence or presence of varying doses of LAB for 1 h and stimulated with 10 ng/mL PDGF-BB for 10 min or 100µM H₂O₂ for 1 min. Then the cells were incubated in the dark for 10 min with DCF-DA. Culture dishes were inspected with a fluorescence microscope imaging system, and the cellular fluorescence was visualized);

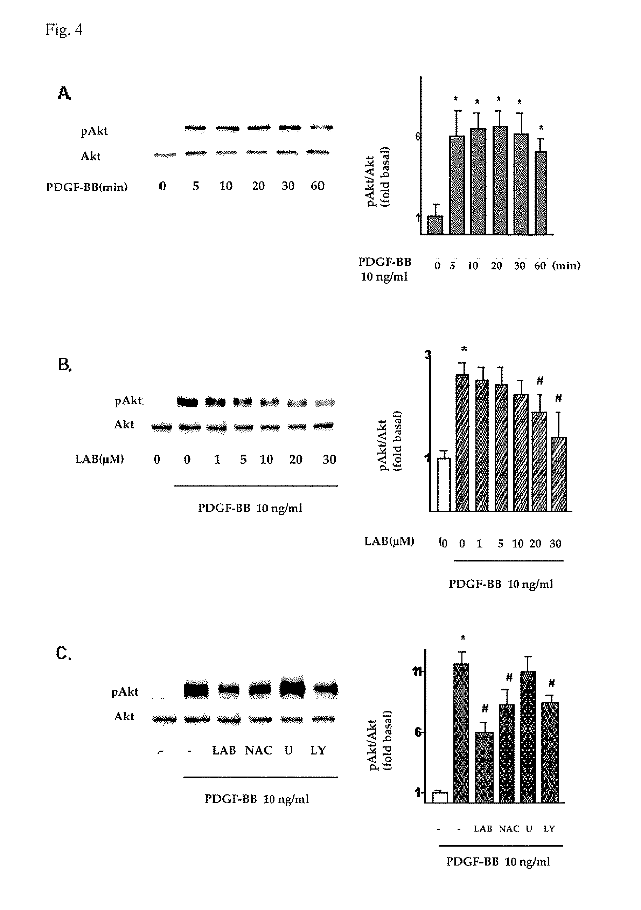

Fig. 4A-4C are graphs showing the effects of LAB on phosphorylation and activity of intracellular Akt induced by PDGF-BB [4A: VSMCs were treated with 10 ng/ml PDGF-BB for time periods ranging from 5 min to 1 h. 4B: Effect of LAB on PDGF-BB-induced Akt activation in VSMCs. VSMCs were pretreated with the indicated concentrations of LAB(1, 5, 10, 20 and 30 µM) for 1 h, then stimulated with 10 ng/mL PDGF-BB for an additional 10 min. 4C: Effect of various inhibitors on PDGF-BB-induced Akt activation in VSMCs. VSMCs were pretreated with 10µM U0126, 10 µM SB203580, 40 µM LY294002 and 20mM NAC and 30µM LAB for 1h, and then stimulated with 10 ng/mL PDGF-BB for 10 min. Values are means ± SD. *, *p*< 0.001 compared with the control; #, *p*< 0.001 compared with the PDGF-BB alone treated group (n=3).];

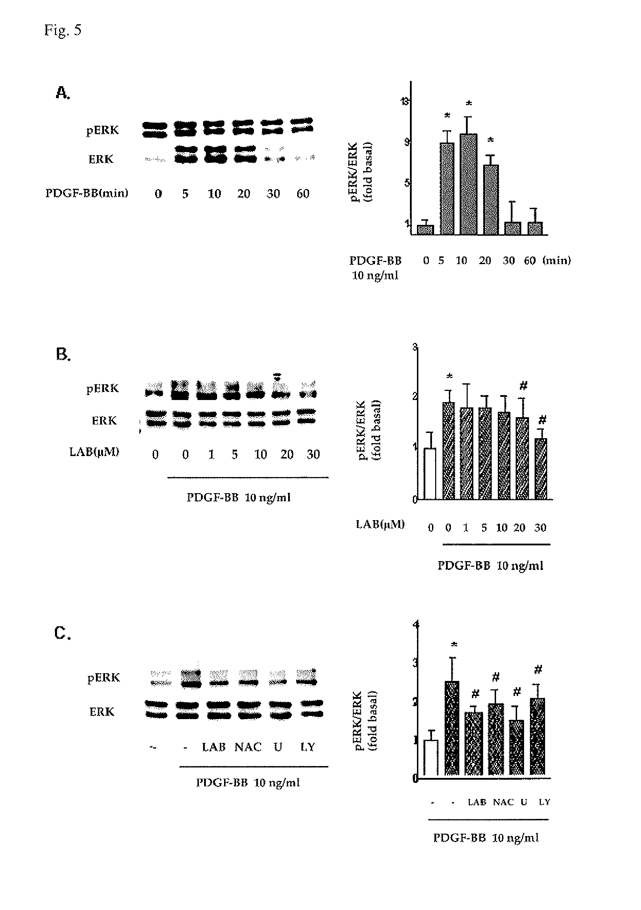

Fig. 5A-5C are graphs showing the effects of LAB on phosphorylation and activity of intracellular ERK1/2 induced by PDGF-BB [5A: PDGF-BB increased ERK 1/2 posphorylation in VSMCs. VSMCs were treated with 10 ng/mL PDGF-BB for time periods ranging from 5 min to 1 h. 5B: Effect of LAB on PDGF-BB-induced ERK 1/2 activation in VSMCs. VSMCs were pretreated with the indicated concentrations of LAB (1, 5, 10, 20 and 30µM) for 1 h, then stimulated with 10ng PDGF-BB for an additional 10 min. 5C: Effect of various inhibitors on PDGF-BB-induced ERK 1/2 activation in VSMCs. VSMCs were pretreated with 10µM U0126, 10µM SB203580, 40µM LY294002 and 20mM NAC and 30µM LAB for 1h, and then stimulated with 10 ng/mL PDGF-BB for 10 min. Values are means ± SD. *, *p*< 0.001 compared with the control ; #, *p*< 0.001 compared with the PDGF-BB alone treated group (n=3).]; and

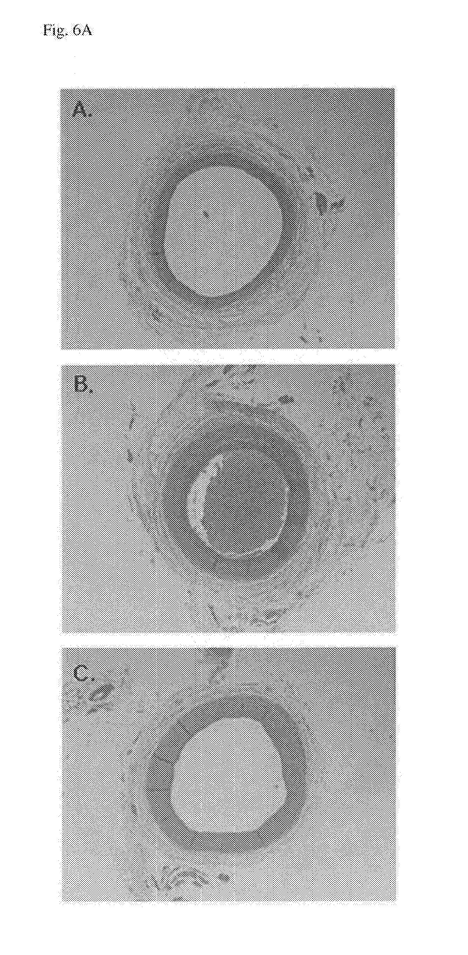

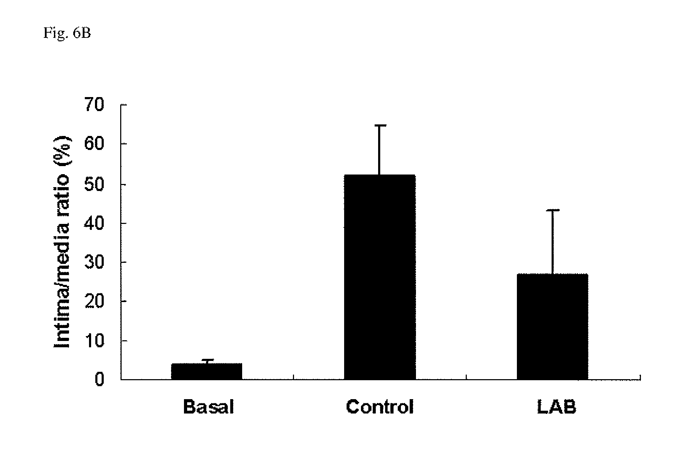

Figs. 6A and 6B show the effects of LAB inhibiting re-stricturization of vessels in an animal model with injured vessels [Representative observations are sections from an uninjured vessel(6A), a balloon-injured vessel(6B), and a LAB-treated balloon injured vessel(6C). Data are also quantified by the neointima/media ratio of common carotid arteries after balloon injury from each group of animal studies(6B). Arrows indicate the elastic laminae over which are the neointimal layers. All pitures were taken at x 200 magnification. Values are means± SD. #, p< 0.05 compared with the basal ; *, p< 0.01 compared with the control (n=8).]

### DETAILED DESCRIPTION OF PREFERRED EMBODIMENTS

In an embodiment, this invention provides a pharmaceutical composition comprising lithospermate B ('LAB' hereinafter) that inhibits proliferation and migration of vascular smooth muscle cells ('VSMC' hereinafter).

In another embodiment, this invention provides a pharmaceutical composition comprising LAB for treating atherosclerosis.

In a further embodiment, this invention provides a health food comprising LAB as an active ingredient for preventing proliferation and migration of VSMC. In particular, this invention provides a pharmaceutical composition comprising LAB isolated from *Salviae miltiorrhiza* as an active ingredient for treating atherosclerosis.

The LAB expressed in the following formula 1 used in the present invention can be isolated from *Salviae miltiorrhiza* or prepared via the conventional organic synthesis.

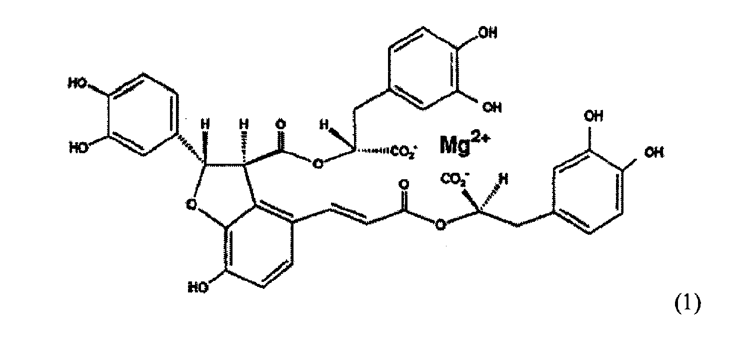

LAB has been shown to have excellent effect in inhibiting proliferation and migration of VSMC. Therefore, LAB or its pharmaceutically acceptable salts can be used to treat atherosclerosis by inhibiting the proliferation and migration of VSMC.

The pharmaceutical composition of the present invention can be administrated orally or parenterally, for example, intravenously, subcutaneously, peritoneally or tropically, and can be used as a conventional type of a pharmaceutical drug.

The pharmaceutical composition of the present invention can be formulated in the form of oral preparations such as tablets, troches, lozenges, hydrophilic or lipophilic suspensions, powders, granules, hard or soft capsules, syrups or elixirs.

For the formulation into tablets or capsules, the pharmaceutical composition of the present invention comprises a binder such as lactose, sucrose, sorbitol, mannitol, starch, amylopectin, cellulose or gelatine; an excipient such as dicalcium phosphate; a disintegrater such as corn starch or sweet potato starch; and a lubricator such as magnesium state, calcium stearate, sodium stearylfumarate, or polyethylene glycol wax.

In case of capsule preparations, they can further comprise a liquid carrier such as fat oil in addition to the above-mentioned substances.

The pharmaceutical composition of the present invention can be administrated parenterally including subcutaneous, intravenous, or intramuscular injections.

To formulate the pharmaceutical composition of the present invention into a preparation for parental administration it is necessary to mix LAB with a stabilizer or a buffer in the presence of water or prepare it into a suspension and then manufactured in the unit dosage form of ampoules or vials.

The effective amount of administration of the active ingredient is 1 - 100 mg/day per 1 kg of an adult patient, preferably 5 - 50 mg/day. It can be administered from one to several times daily upon consultation with a physician or a pharmacist, preferably 2 -3 times daily.

In the present invention, health food refers to those where the above active ingredient is added to food substance such as drinks, teas, spices, gums, snacks, etc., or is manufactured in the form of capsules, powders, and suspensions, dietary intake of which results in a certain health effect but does not exhibit any known adverse effects even when administered for a long period of time unlike in the case of administration of normal medicinal drugs.

This invention is explained in more detail based on the following Examples, however, they should not be construed as limiting the scope of this invention.

### Examples

**Reference Example 1 : Separation of Vascular Smooth Muscle Cells of a Sprague-Dawley and Their Culture**

Vascular smooth muscle cells ('VSMC' hereinafter) were separated from thoracic aorta of a Sprague-Dawley weighted about 100-150g by using collagenase digestion method and then cultured them according to the method disclosed in previous studies [Gennaro G, Menard C, Giasson E, et al. Role of p44/p42 MAP kinase in the age-dependent increase in vascular smooth muscle cell proliferation and neointimal formation. Arterioscler Thromb Vasc Biol. Feb 1 2003;23(2):204-210.; Hamaguchi A, Kim S, Izumi Y, et al. Contribution of extracellular signal-regulated kinase to angiotensin II-induced transforming growth factor-betal expression in vascular smooth muscle cells. Hypertension. Jul 1999;34(1):126-131].

The VSMC was cultured in a waterbath kept at 37 °C with the condition of 95% of humidity and 5% CO₂ using Dulbecco's modified Eagle's medium(DMEM) containing 10% (v/v) heat inactivated fetal bovine serum, 100 unit/mL penicillin, and 100 g/mL streptomycin as a culture medium.

The VSMC was treated in a culture medium not containing serum for 24 hrs prior to treatment with PDGF-BB, and the reaction according to the presence or absence of PDGF-BB was analyzed after pretreating with LAB or other reagents. PDGF-BB(Sigma-Aldrich, St. Louis, MO, USA) used was 10 ng/mL, and LAB was used by varying its concentration up to 30µM and there was no adverse reaction observed even in the highest concentration.

**Reference Example 2 : Measurement of Cell Proliferation Capability**

LDH Assay: LDH assay: VSMC was stabilized by adding them into DMEM (400 µL) containing 10% FBS in each of the 12 well plates (1 x 10⁴ cells/well) and placed for 24 hrs without serum prior to treatment with other reagents to stop cell proliferation and differentiation. Then, the cells were pretreated with LAB (1, 5, 10, 20, 30µM), U0126(1,4-diamino-2,3-dyacyano-1,4-bis (2-amino-phenylthio) butandiene; a MEK inhibitor) 10 µM, LY294002 ((2-(4-morpholinyl)-8-phenyl)-4H-1-benzopyran-4-one; a PI3K inhibitor) 40 µM, or 20 mM NAC(N-acetylcysteine) for 1 hr, and then treated with PDGF-BB 10 ng/mL for 24hrs. The cells were then washed twice with PBS, lysed by using 0.5% Triton-X100, and then the activity of lactate dehydronase(LDH) was measured by using CytoTox-ONETM assay kit (Promega, Madison, WI, USA) with fluorescence at 560/590 nm.

MTT Assay : VSMC was prepared in the 12 well plates as in the above LDH Assay and treated with reagent. Colorimetric method, which measures the level of conversion of the yellow water-soluble methyl-thiazo-etetrazolium (MTT)[Sigma-Aldrich, St. Louis, MO, USA] into the bluish non-water-soluble formazan by succinyl dehydrogenase of mitochondria. This method can measure the level of metabolism of live cells and thus the result can reflect the number of live cells. MTT in the amount of 0.5 g/mL was cultured in a waterbath with constant temperature and humidity for 4 hrs, added with 400 L of dimethylsulfoxide(DMSO) to dissolve formazan crystal, and then optical densitywas measured at 570 nm by using ELISA plate reader(Spectra MAX3 340, Molecular devices Corp, Sunnyvale CA, USA).

**Reference Example 3 : Measurement of Cell Migration**

The migration of VSMC with 8 µm of pores was measured by using modified Boyden chamber method[Brown C, Pan X, Hassid A. Nitric oxide and C-type atrial natriuretic peptide stimulate primary aortic smooth muscle cell migration via a cGMP-dependent mechanism: relationship to microfilament dissociation and altered cell morphology. Circ Res. Apr 2 1999;84(6):655-667], which utilizes collagen-coated transwell tissue culture inserts consisting of polycarbonate membrane. 3.0 x 10⁶ cells/mL of the above vascular smooth muscle cells were allowed to float in a culture medium without serum, treated with LAB with various concentrations and then cultured at 37°C for 30 min. Then, the cells were treated with either 0.5 mL DMEM without serum or 0.5 mL DMEM without serum along with PDGF-BB 10 ng/mL and then placed in the lower part of a chamber. 100 µL of the folating materials of the above cells (final 3.0 x 10⁵ cells/well) was added to the upper part of the chamber and cultured at 37°C for 16 hrs. The cells were then fixed, dyed with hematoxylin, and filters were dried and developed.

**Reference Example 4 : Measurement of the Amount of Reactive Oxygen Species**

The amount of reactive oxygen species in cells was measured as described in the previous report [Hamaguchi A, Kim S, Izumi Y, et al. Contribution of extracellular signal-regulated kinase to angiotensin II-induced transforming growth factor-beta1 expression in vascular smooth muscle cells. Hypertension. Jul 1999;34(1):126-131]. VSMC was cultured in a culture medium without serum for 24 hrs to stop cell proliferation and then pretreated with LAB with various concentrations of 1, 5, 10, 20, 30 µM for 1 hr. The cells were treated with either 10 ng/mL of PDGF-BB for 10 min or with 100 µM H₂O₂ for 1 min and then cultured in the presence of 5-(and-6)-chloromethyl-2',7'-dichlorohydrofluorescein diacetate(CM-H2DCF-DA: Molecular Probes Inc., Eurogene, OR, USA) in the dark room for 10 min and cultured cells and generation of reactive oxygen species were observed by using fluorescent microscope.

**Reference Example 5 : Western Blot Analysis**

VSMC was washed with PBS and then dissolved in lysis buffer(25mM Tris HCl, pH 7.4, 25mM NaCl, 25mM NaF, 25mM sodium pyrophosphate, 1mM sodium vanadate, 2.5mM EDTA, 2.5mM EGTA, 0.05% (wt/vol) Triton X-100, 0.5% (wt/vol) SDS, 0.5% (wt/vol) deoxycholate, 0.5% (wt/vol) Nonidet P-40, 5 g/mL leupeptin, 5 g/mL aprotinin, and 1mM PMSF). The cells were collected and centrifuged at 13,000 rpm for 15 min and the protein concentration of the resulting supernatant was measured by Bradford assay. The proteins were denatured and were subjected to SDS-PAGE and then blotted onto nitrocellulose membranes. The membranes were placed in 5%(w/v) non-fat dry milk in TBS with 0.1 % Tween20(TBS-T) and then placed at room temperature for 3 hrs. The blots were placed at 4 °C along with anti-ERK1/2 or anti-Akt antibodies(Cell signaling Technologh, Berely. MA, USA) for 16 hrs. The membranes were washed 4 times with TBS-T, added with anti-rabbit IgG antibody conjugated with horseradish peroxidase(1:2,000 diluent) and then placed at room temperature for 1 hr. The immunoreactive bands detected by ECL reagents were developed by Hyperfilm-ECL(Amersham Life Science, Little Chalfont, UK). Thebands showing positive responses were quantitated by densitometry and then compared with that of control.

**Reference Example 6 : Animal Model with Atheroscleosis**

Two French Forgarty embolectomy catheters were inserted into white male Sprague-Dawley (300 g) through external carotid arteries, and blood vessels were intentionally damaged by moving catheters, which were swollen by adding a physiological saline solution, 3 times vertically within the common carotid thereby expanding blood vessels while shedding off the endothelial cells. A total of 30 rats were divided into 3 groups (10 rats/group) and used in this experiment: group 1 was not treated; group 2 was damaged with their blood vessels; and group 3 was treated with LAB and then damaged with their blood vessels. LAB (10 mg/kg/day) was injected intravenously for a period of 30 days (i.e., from 2 days prior to the blood vessel damage to 28 days after the blood vessel damage). The rats were sacrificed on the 28^{th} day after the blood vessel damage by ablating carotid artery and it was fixed with 4% formaldehyde and then dyed with hematoxylin-eosin. The mid parts of the damaged blood vessel were cut into 2-4 parts and analyzed via computerized morphometry(SCN Image). The portion of the newly generated part (neointimal area, N) in the mid parts were indicated in percentage (N/M %).

**Example 1 : Influence of LAB on Proliferation of VSMC induced by PDGF-BB**

To investigate whether LAB decreases the proliferation of VSMC induced by PDGF-BB an experiment was performed as follows. The VSMC was placed in a culture medium not containing serum for 24 hrs to inhibit cell proliferation, and then pretreated with LAB with various concentrations of 1, 5, 10, 20, 30µM and treated with PDGF-BB for 24 hrs. The extent of VSMC proliferation was compared with that of control as stated above via LDH and MTT assays (Fig.1). As shown in Fig.1, VSMC proliferation was decreased in proportion to the concentration of LAB. The concentration of LAB was used up to 30µM but there was no cytotoxic activities observed (data not shown) and therefore it was concluded that the above decrease in VSMC proliferation was not due to the cytotoxicity of LAB.

To investigate the reaction mechanism of LAB at the molecular level and its signal transduction pathway, several inhibitors of VSMC proliferation such as U0126 (20µM, MEK inhibitor), LY294002(40 µM, PI3K inhibitor), and NAC(20 mM), which is involved in intracellular antioxidation (Fig. 1B), were tested. However, SB202190(p38 MAPK inhibitor) and JNK inhibitor I(JNK inhibitor) showed no distinct inhibitory activity against VSMC proliferation induced by PDGF-BB (data not shown).

**Example 2 : Influence of LAB on Migration of VSMC induced by PDGF-BB**

It is known that LAB plays an important role in proliferation of VSMC as well as development of atherosclerosis. To investigate whether LAB decreases the migration of VSMC induced by PDGF-BB, an experiment was performed as follows by using the aforementioned modified Boyden chamber method. As shown in Fig.2, the migration of VSMC was increased by PDGF-BB about 60%. However, when it was pretreated with LAB, the migration was decreased in proportion to the concentration of LAB.

**Example 3 : Influence of LAB on Production of Reactive Oxygen Species present in intracellular VSMC induced by PDGF-BB**

It has been known that reactive oxygen species serve as an intermediate mediator for cytokines or other growth factors which are involved in signal transduction pathways. PDGF-BB has been also well known that it can expedite the generation of intracellular reactive oxygen species. Therefore, to investigate whether LAB inhibits the production of VSMC induced by PDGF-BB an experiment was performed as follows.

First, the amount of intracellular reactive oxygen species generated by PDGF-BB was measured by using peroxide-sensitive fluorophore DCF. As shown in Fig. 3A (B-1), the amount of intracellular reactive oxygen species generated by PDGF-BB evaluated with DCF fluorescence was 2.7 times greater. DCF fluorescence due to PDGF-BB was temporarily increased within 5 min and maintained for about 20 min. In this experiment, LAB was treated with PDGF-BB for 10 min. When LAB was pretreated, the amount of DCF fluorescence was decreased as compared to when it was treated only with PDGF-BB [Fig. 3A(A)]. The amount of intracellular reactive oxygen species generated by PDGF-BB PDGF-BB was decreased in proportion to the concentration of LAB[Fig. 3B(B)]. Further, LAB decreased the activity of hydrogen peroxide, which increases the generation of intracellular reactive oxygen species, in proportion to its concentration (Fig. 3A(C)).

**Example 4 : LAB's Mechanism of Inhibiting VSMC Proliferation induced by PDGF-BB**

It is not yet clearly known how VSMC grows. The phosphorylation and dephosphorylation of protein kinase play crucial roles in various kinds of protein syntheses [Servant MJ, Giasson E, Meloche S. Inhibition of growth factor-induced protein synthesis by a selective MEK inhibitor in aortic smooth muscle cells. J Biol Chem. Jul 5 1996;271(27):16047-16052.].

In Fig. 1B, it is shown that both U0126 (20 µM) and LY294002 (40 µM) inhibited activities of LDH and MTT induced by PDGF-BB. As a way to investigate the signal transduction pathway involved in VSMC proliferation induced by PDGF-BB, an experiment was conducted to examine whether PDGF-BB activates a relatively lower signal transduction pathway such as ERK1/2 and Akt. As shown in Figs. 4A and 5A, there were increases in activity level when treated with PDGF-BB; that is, the activity of ERK1/2 was increased 11 folds while that of Akt increased 7 folds. However, it was shown that LAB inhibits phosphorylation of ERK1/2 and Akt due to PDGF-BB in proportion to its concentration, and it was most effective at 30 µM [Figs. 4B, 5B].

To examine whether the decrease in intracellular reactive oxygen species by LAB is related with the inhibition of VSMC proliferation, LAB was pretreated with an antioxidizing agent before treating with PDGF-BB and the inhibitory level of VSMC proliferation. The results showed that LAB decreased 0.8 fold and NAC decreased 0.6 fold in the event of ERK1/2 phosphorylation; whereas LAB decreased 8 folds and NAC decreased 3.4 folds in the event of Akt phosphorylation[Figs. 4C, 5C].

Then, the relationship between the phosphorylation of ERK1/2 and Akt due to PDGF-BB. As shown in the Western blot analysis, the phosphorylation of Akt was decreased only by LY294002 (3.5 folds), whereas ERK1/2 phosphorylation was decreased by both LY294002 and U0126. Therefore, it is speculated that Akt phosphorylation due to PDGF-BB is in an upper signal transduction pathway than ERK1/2 phosphorylation in its hierarchy [Figs. 4C, 5C].

**Example 5 : Effect of LAB on neointimal hyperplasia in an Animal Model**

White Sprague-Dawley rats were damaged by using percutaneous transluminal angioplastyas ('PTA') as stated above. LAB in the amount of 10 mg/kg/day or placebo was administered to the above rats starting from 2 days prior to 'PTA' surgery. On the 2oth day after the surgery the rats were sacrificed and the Intima-Media Thickness ('IMT') was measured. The rats were compared with undamaged rats and the result shows that the increase in 'IMT' was much low in LAB-treated rats than in those treated with placebo.

**Example 6 : Test of Acute Toxicity due to Oral Administration in SD Rats**

Acute toxicity test was performed using 6 week old SPF SD rats. The SD rats were divided two per each group and each rat was orally administered once with LAB in the amount of 1 g/kg/1mL, which was suspended in 0.5% methylcellulose. After the above administration, the rats were subjected to examination with regard to survival, clinical symptoms, change in body weight, and the like and also hematological examination, biochemical blood test. Further, autopsies were performed to observe abnormalities in abdominal organs. The results showed that all experimental rats survived and also there were observed no abnormal clinical symptoms, no toxicities and other abnormalities in the above-mentioned tests. Therefore, it was concluded that the tested compound has no toxicity to rats up to 500 mg/kg and the LD₅₀ was greater than 500 mg/kg thus proving that the compound is a safe material.

**Preparation Example 1 : Syrup Preparation**

A syrup comprising LAB of the present invention in the amount of 2%(wt/vol.) as an active ingredient was prepared as follows.

LAB, saccharin, and sugar were dissolved in 80 g of warm water. The solution was cooled down and added with a mixture consisting of glycerine, saccharin, flavour, ethanol, sorbic acid and distilled water, which was then added with water to a final volume of 100 mL.

The components of the above syrup preparation is as follows:

| | |
|---|---|
| LAB | 2 g |
| saccharin | 0.8 g |
| sugar | 25.4 g |
| glycerin | 8.0 g |
| flavor | 0.04 g |
| ethanol | 4.0 g |
| sorbic acid | 0.4 g |
| distilled water | adequate |

**Preparation Example 2 : Tablet Preparation**

A tablet comprising LAB in the amount of 15 mg was prepared as follows.

LAB in the amount of 250 g was mixed with 175.9 g of lactose, 180 g of potato starch, and 32 g of colloidal silicic acid. The mixture was added with 10% gelatine solution, then pulverized and then passed through a mesh of 14 in size. The resultant was dried and added with 160 g of potato starch, 50 g of talc and 5 g of magnesium stearate to finally obtain a tablet.

**Preparation Example 3 : Injection Preparation**

An injection preparation comprising LAB in the amount of 10 mg was prepared as follows.

One gram of LAB, 0.6 g of sodium chloride and 0.1 g of ascorbic acid were dissolved in distilled water to a final volume of 100 mL. The mixture was added into a vial and sterilized by heating at 20 °C for 30 min.

**Preparation Example 4 : Drink Preparation**

An injection preparation comprising LAB in the amount of 10 mg was prepared as follows.

LAB in the amount of 500 mg was dissolved in an adequate amount of water and then added with an adequate amount of vitamin C as a supplementary component; with an adequate amount of citric acid, sodium citrate, and with an adequate amount of oligosaccharide as a sweetener; with an adequate amount of sodium benzoate as a preservative; and then added with water to a final volume of 100 mL to finally obtain a drink preparation. Here, taurine, Myo-inositol, folic acid, or pantothenic acid can be added alone or in combination.

**Industrial Applicability**

As stated above, LAB of the present invention can inhibit the proliferation and migration of VSMC thereby providing a therapeutic effect for treating atherosclerosis. Therefore, the LAB of the present invention can be usefully used as a functional food and a pharmaceutical drug without any adverse effects.

The invention has been described in detail with reference to preferred embodiments thereof. However, it will be appreciated that those skilled in the art, upon consideration of the disclosure, may make modifications and improvements within the scope and spirit of the invention.

## Claims

1. A pharmaceutical composition comprising lithospermate B as an active ingredient for inhibiting proliferation and migration of vascular muscle cells.

2. A pharmaceutical composition comprising lithospermate B as an active ingredient for treating atherosclerosis.

3. The pharmaceutical composition according to claim 1 or claim 2, wherein said composition is formulated for oral or parenteral administration.

4. A health food comprising lithospermate B as an active ingredient for preventing proliferation and migration of vascular smooth muscle cells.
